# EUROPÄISCHE PATENTSCHRIFT

(11) **EP 3 448 322 B1**
(45) Veröffentlichungstag und Bekanntmachung des Hinweises auf die Patenterteilung: **27.08.2025**
(21) Anmeldenummer: 17718940.4
(22) Anmeldetag: 25.04.2017
(51) Int. Cl.: A61F 2/78, A43B 11/00, A61F 5/01, A61F 5/058

(54) **HÜLLKÖRPER**
ENVELOPING BODY
CORPS ENVELOPPANT

(30) Priorität: 26.04.2016 DE 102016107670
(43) Veröffentlichungstag der Anmeldung: 06.03.2019
(73) Patentinhaber: Ottobock SE & Co. KGaA, 37115 Duderstadt (DE)
(72) Erfinder: FINKE, Lars Benjamin, 37136 Landolfshausen (DE)
(74) Vertreter: Gramm, Lins & Partner Patent- und Rechtsanwälte PartGmbB
(86) Internationale Anmeldenummer: PCT/EP2017/059729
(87) Internationale Veröffentlichungsnummer: WO 2017/186680

(56) Entgegenhaltungen:
- WO-A1-2015/083810
- DE-C- 917 687
- SU-A1- 731 963
- US-A- 1 057 562
- US-A1- 2016 270 479

## Beschreibung

Die Erfindung betrifft einen Hüllkörper zur zumindest teilweisen Umhüllung einer Gliedmaße mit einem eingeschlossenen Volumen und einem Anschluss zum Zuführen und Abführen von Fluid in das Volumen oder aus dem Volumen, wobei der Hüllkörper einen Innenumfang und einen Außenumfang ausbildet. Der Hüllkörper ist zur Anlage entweder an einen Stumpf oder aber an eine Gliedmaße vorgesehen.

Zwischen einem Prothesenschaft und einem Stumpf einer Gliedmaße ist zur Polsterung des Stumpfes, zum Ausgleichen von Unebenheiten der Schaftoberfläche sowie zum Herstellen einer Verbindung zwischen dem Stumpf und dem Prothesenschaft ein Liner vorgesehen, der über den Stumpf gezogen wird. Der Liner haftet in der Regel an der Hautoberfläche des Stumpfes und kann an seinem distalen Ende Verriegelungselemente aufweisen, beispielsweise einen Pin, der mit einer korrespondierenden mechanischen Verriegelungseinrichtung in dem Prothesenschaft wechselwirkt. Im Rahmen der Vakuumschafttechnologie kann zwischen dem Liner und dem Prothesenschaft ein abgedichtetes Volumen ausgebildet werden, aus dem über eine Pumpe oder ein Ventil Luft herausgefördert wird. Über den Unterdruck wird erreicht, dass der Prothesenschaft an dem Prothesenliner gehalten wird.

Die Prothesenliner bestehen in der Regel aus einem Elastomerwerkstoff, beispielweise Silikon, der eine hohe Haftfähigkeit an dem Stumpf aufweist. Zum Anlegen wird der Liner aufgerollt, das distale, vorzugsweise geschlossene Ende des Prothesenliners wird auf das Stumpfende aufgelegt und der Liner dann über den Stumpf abgerollt. Regelmäßig weist der Liner einen geringeren Innendurchmesser als der Außendurchmesser des Stumpfes auf, um einen vergleichsweise festen Sitz an dem Stumpf zu ermöglichen.

Problematisch hierbei ist, dass eine ausreichende Dehnung des Materials erfolgen muss, um den Prothesenliner anliegen zu können. Dies ist insbesondere für Patienten mit weiteren körperlichen Einschränkungen schwierig.

Aus der US 5,387,245 A ist ein Prothesenliner mit einem Innenliner und einem Außenliner aus einem Elastomermaterial bekannt. Der Innenliner und der Außenliner sind miteinander verklebt, wobei zur Ausbildung einer Blase zwischen dem Innenliner und dem Außenliner ein Bereich unverklebt ist. Ein Ventil ist der Blase zugeordnet und ermöglicht es, Luft in die Blase einzuführen oder abzulassen, um Volumenschwankungen des Stumpfes ausgleichen zu können.

Weiterhin existieren aus dem Stand der Technik Bandagen oder Manschetten, die um eine Gliedmaße herum angelegt werden, um einen umfänglichen Druck auf die Gliedmaße auszuüben. Solche Bandagen oder Manschetten sind bevorzugt aus einem Gewebe oder einem Neopren hergestellt. Zum Anlegen müssen diese Hüllkörper, die eine proximale und eine distale Öffnung aufweisen, ebenfalls gedehnt werden.

Die DE 917 687 C betrifft eine Fassung für einen Beinstumpf. Die Fassung ist doppelwandig und aus einem biegsamen, elastischen Material hergestellt. Die Wände bilden einen abgeschlossenen, luftdichten Hohlraum, der mit einem Ventil verbunden ist, über das Luft in den Hohlraum eingelassen oder abgelassen werden kann. Eine Reihe von Geweben ist vorgesehen, die sich über den Hohlraum spannen und den Hohlraum in eine Anzahl von miteinander verbundenen Abteilungen aufteilen. Die Gewebe sind in Längsrichtung und in Umfangsrichtung angeordnet, um ein Netzwerk von Abteilungen zu bilden. Die Gewebe können sich in Längsrichtung und kreisförmig ausdehnen.

Die US 1 057 562 A beschreibt eine Stumpfabstützung bestehend aus einem rechteckigen aufblasbaren Kissen mit einer größeren Länge als der Umfang des Beines, so dass das aufblasbare Kissen überlappend um den Stumpf herum gelegt werden kann. Das Kissen erstreckt sich über und stützt sich auf einer oberen Kante eines Prothesenschaftes ab und ist im Bereich der oberen Kante mit einem Ventil ausgestattet.

Die SU 731 963 betrifft eine Prothese für eine untere Gliedmaße. In einem starren Prothesenschaft sind aufblasbare Schläuche zickzackartig angeordnet, um eine Anpassung an den Stumpf zu erleichtern.

Die WO 2015/083810 A1 betrifft einen Schuh, der leicht anzuziehen und leicht auszuziehen sein soll. Er soll billig, leicht, komfortabel, gesundheitsfördernd und längeneinstellbar sein. Dies wird dadurch erreicht, dass die Formen einer äußeren Schicht und innenliegender Volumina korrespondierend zu verschiedenen Körperteilen angeordnet sind. Sowohl die äußere Schicht als auch die Volumina bestehen aus einem dehnbaren Material, die hauptsächlich mit der Innenseite der äußeren Schicht verbunden sind. Über Ventile können die Volumina mit Luft befüllt oder entleert werden. Wird Luft in die Volumina eingeleitet, dehnt sich der gesamte Schuh aufgrund der Flexibilität aus und erleichtert das Anziehen des Schuhs. Nachdem der Fuß eingeführt wurde, werden die Ventile geöffnet und Luft wird durch das Zusammenziehen der Volumina herausgedrückt. Die Volumina zusammen mit der äußeren Schicht des Schuhs treten in einen engen Kontakt mit dem Fuß, so dass der Schuh ohne Rutschen getragen werden kann.

Aufgabe der vorliegenden Erfindung ist es, einen Hüllkörper bereitzustellen, der einfach anzulegen ist und dessen Passform individuell an den Nutzer anpassbar ist.

Erfindungsgemäß wird diese Aufgabe durch einen Hüllkörper mit den Merkmalen des Hauptanspruches gelöst. Vorteilhafte Ausgestaltungen und Weiterbildungen der Erfindung sind in den Unteransprüchen, der Beschreibung sowie den Figuren offenbart.

Der erfindungsgemäße Hüllkörper zur zumindest teilweisen Umhüllung einer Gliedmaße mit einem eingeschlossenen Volumen und einem Anschluss zum Zuführen und Abführen von Fluid in das Volumen oder aus dem Volumen, wobei der Hüllkörper einen Innenumfang und einen Außenumfang ausbildet, sieht vor, dass der Hüllkörper als Prothesenliner ausgebildet ist, einen Aufnahmeraum für einen Stumpf ausbildet und der Innenumfang des Prothesenliners sich bei einer Druckerhöhung des Volumens vergrößert. Wird Fluid aus dem Volumen abgelassen, verringert sich der Innenumfang des Hüllkörpers aufgrund einer bevorzugt durch das Material des Hüllkörpers aufgebrachten elastischen Rückstellkraft. Dadurch ist es möglich, durch eine Druckerhöhung innerhalb des Hüllkörpers eine Umfangserweiterung des Innenraumes der Hüllkörpers zu erreichen, so dass der Hüllkörper leicht angelegt werden kann. Nachdem der Hüllkörper in der gewünschten Position ist, also entweder über einen Stumpf oder um eine Gliedmaße herum angelegt ist, kann das Fluid aus dem eingeschlossenen Volumen abgelassen werden, wodurch sich der Innendruck verringert und sich der Innenumfang an die Kontur des Stumpfes oder der aufgenommenen Gliedmaße anlegt und anpasst, wobei sich das Volumen des Aufnahmeraumes bei einer Druckminderung verringert und die Innenwand im angelegten Zustand eine Anpresskraft auf den Stumpf ausübt, wobei die maximale Anpresskraft erreicht ist, wenn das Volumen minimal ist. Das Maß des Anpressdruckes kann durch die Wahl des Innendruckes variiert werden. Je geringer der Innendruck ist, desto höher ist der Anpressdruck an die Gliedmaße. Der maximale Anpressdruck wird durch die Materialeigenschaften des Hüllkörpers und dessen Abmessungen bestimmt. Je größer das Untermaß des Hüllkörpers mit einem nicht mit Druck beaufschlagten Volumen ist, desto größer ist die Vorspannung gegenüber dem Stumpf oder der Gliedmaße. Der Hüllkörper weist eine Innenwand und eine Außenwand auf, die das mit Fluid befüllbare Volumen einschließen. Als Fluid wird vorzugsweise ein kompressives Medium, insbesondere die Umgebungsluft verwendet. Die Innenwand und die Außenwand können beispielsweise in ihren distalen und proximalen Endbereichen miteinander fluiddicht verbunden sein, so dass bei einer Ausgestaltung als Liner sich das Volumen hülsenförmig ausbildet. Das Volumen ist dann im Bereich der Seitenwand, die sich von einem distalen, geschlossenen Endbereich bis zu der proximalen Einstiegsöffnung erstreckt, angeordnet. Die Innenwand und die Außenwand sind bevorzugt aus dem gleichen Material hergestellt, zumindest haben sie gleiche elastische Eigenschaften. Zwischen der Innenwand und der Außenwand sind zugkraftübertragende Elemente angeordnet, wobei die zugkraftübertragenden Elemente zugstarr oder elastisch ausgebildet sein können. Unter zugstarren Elementen sind solche Elemente zu verstehen, die bei einer Druckerhöhung innerhalb des Volumens keine oder eine geringere Dehnung als die Innenwand und/oder die Außenwand aufweisen. Die zugkraftübertragenden Elemente können als Gurte, Stege oder Stifte ausgebildet sein und entweder einstückig mit den Wänden ausgebildet oder separat daran befestigt sein.

Zumindest die Innenwand des Hüllkörpers kann elastisch ausgebildet sein, bevorzugt sind sowohl die Innenwand als auch die Außenwand elastisch ausgebildet, um ein Aufweiten beider Wände zu ermöglichen. Es besteht auch die Möglichkeit, dass die Außenwand elastisch und die Innenwand nur teilweise elastisch oder auch aus einem gefalteten oder gerafften, inelastischen Material besteht. Unter inelastisch werden Materialien verstanden, die sich bei den im jeweiligen Anwendungsgebiet üblichen Drücken und Kräften nicht oder nur unwesentlich in ihrer Länge verändern. Neben einer vollständigen elastischen Ausgestaltung, beispielsweise durch Ausbildung der jeweiligen Wand vollständig aus einem Elastomer, können auch nur bereichsweise elastische Bereiche in der Innenwand und/oder Außenwand vorgesehen sein, um eine Vergrößerung des Innenumfangs des Hüllkörpers bei einer Druckerhöhung zu ermöglichen. Ebenfalls ist es vorgesehen, dass die Innenwand und/oder die Außenwand des Hüllkörpers faltbar ausgebildet sind, so dass sich durch das Auffalten der jeweiligen Wand eine Vergrößerung des Umfanges einstellt. Die Faltung ist mit einer Rückstellkraft beaufschlagt, so dass nach der Verringerung des Innendruckes einer korrespondierende Verringerung des Innenumfanges eintreten kann. Die Faltung kann beispielsweise zieharmonikaartig ausgebildet sein. Die Innenwand und/oder Außenwand können somit faltbar und/oder elastisch ausgebildet sein.

Eine weitere Möglichkeit zur Aufweitung des Innenumfang des Hüllkörpers besteht darin, dass dieser zumindest einen Hohlraum aufweist, der zumindest teilumfänglich um den Innenumfang herumgelegt ist, wobei der Hohlraum das befüllbare Volumen umgibt. Durch die teilumfängliche Anordnung des Hohlraumes um den Innenumfang herum ergibt sich bei einer Druckerhöhung des Hohlraumes aufgrund der vorhandenen Krümmung im Ausgangszustand die Tendenz, dass sich der Hohlraum begradigt, so dass insgesamt eine Aufweitung des Innenumfanges erfolgt. Es können mehrere, teilumfänglich um den Innenumfang herum gelegte Hohlräume in Längserstreckung des Hüllkörpers hintereinander angeordnet sein. Die Hohlräume können in einer gemeinsamen Orientierung oder in wechselseitigen Orientierungen ausgebildet sein oder bereichsweise in einer und der anderen Orientierung um den Innenumfang herum angeordnet sein. Sofern nur eine teilumfängliche Ausgestaltung der Hohlräume vorhanden ist, kann beispielsweise über eine gemeinsame Versorgungsleitung jeder Hohlraum oder jeder Hohlraumabschnitt mit dem Fluid versorgt werden. Ebenso wird gemeinsam durch diese Versorgungsleitung das Fluid aus den Hohlräumen oder Hohlraumabschnitten herausgeleitet. Der Hohlraum kann kreisförmig, also nahezu vollständig um den Innenumfang herumgelegt sein. Darüber hinaus besteht die Möglichkeit, dass der Hohlraum wendelförmig um den Innenumfang herumgelegt ist, nach Art einer Wendelfeder oder Spiralfeder, wobei sich durch Erhöhen des Innendruckes innerhalb des Hohlraumes der Durchmesser der Wände vergrößert und eine Innenumfangserhöhung eintritt.

Bevorzugt ist der Hohlraum oder der jeweilige Hohlraumabschnitt schlauchartig ausgebildet. Sofern der Hohlraum zwischen einer Innenwand und einer Außenwand befestigt ist oder aber auch nur an einer Innenwand oder einer Außenwand angeordnet ist, muss der Hohlraum selbst keine elastischen Rückstellkräfte aufweisen, diese können vielmehr durch die jeweilige Wand ausgebildet werden. Der Hohlraum oder Schlauch kann an der Innenwand und/oder Außenwand festgelegt sein, ebenso ist es möglich, dass der Hohlraum zwischen der Innenwand und der Außenwand eingelegt ist und durch die Wände selbst innerhalb des Hüllkörpers positioniert wird. Bevorzugt ist der schlauchartige Hohlraum als elastischer Schlauch ausgebildet, wobei die Elastizität bei dem Schlauchdurchmesser in Verbindung mit dem von dem Schlauch umgebenden Innenumfang vorhanden sein kann, so dass sich Schlauchdurchmesser und Innenumfang bei einer Druckerhöhung vergrößern. Ebenso ist es möglich, dass die Elastizität im Wesentlichen nur bei dem von dem Schlauch umgebenden Innenumfang wirksam ist, sich der Innenumfang bei einer Druckerhöhung also vergrößert, ohne dass sich der Schlauchdurchmesser wesentlich verändert.

Der Hüllkörper kann zumindest teilweise eine haftende Innenoberfläche aufweisen, um eine sichere Positionierung des angelegten Hüllkörpers an dem Stumpf der Gliedmaße sicherzustellen. Bevorzugt erstreckt sich das Volumen über die gesamte Axialerstreckung des Hüllkörpers, entweder durch die Ausgestaltung des Volumens zwischen zwei Wänden oder aber durch eine entsprechende Anordnung von in Axialrichtung hintereinander angeordneten Hohlkörpern über die gesamte Länge oder nahezu die gesamte Länge des Hüllkörpers.

. Bei der Ausgestaltung als Prothesenliner dient der Hüllkörper als Interface zwischen dem Stumpf und einem Prothesenschaft.

Nachfolgend werden Ausführungsbeispiele der Erfindung anhand der beigefügten Figuren näher erläutert. Es zeigen:
- Figur 1 -: eine schematische Darstellung eines Hüllköpers in Gestalt eines Prothesenliners in zwei Zuständen;
- Figur 2 -: eine schematische Darstellung eines Hüllkörpers mit einem wendelförmigen Hohlkörper;
- Figur 3 -: eine schematische Darstellung der Funktionsweise eines Hohlkörpers mit einem teilumfänglichen Hohlkörper;
- Figur 4 -: eine Darstellung eines Hüllkörpers als Prothesenliner in perspektivischer Ansicht;
- Figur 5 -: eine schematische Darstellung einer Bandage, die nicht von der Erfindung umfasst ist; sowie
- Figur 6 -: eine schematische Darstellung eines Hüllkörpers in Gestalt eines Schuhs, der nicht von der Erfindung umfasst ist.

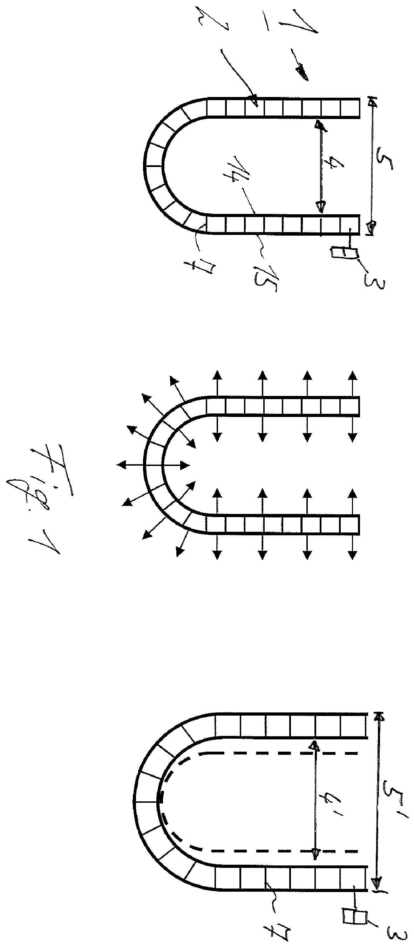

Figur 1 zeigt eine schematische Darstellung eines orthopädietechnischen Hüllkörpers 1 in Gestalt eines Prothesenliners als grundsätzliche Wirkungsweise der Erfindung. Der Hüllkörper 1 weist eine Innenwand 14 und eine Außenwand 15 auf, die zwischen sich ein Volumen 2 einschließen. Der Hüllkörper 1 ist somit doppelwandig ausgebildet und weist einen Anschluss 3 mit einem Ventil auf, um das Volumen 2 zwischen der Innenwand 14 und der Außenwand 15 mit einem Fluid zu befüllen oder Fluid aus dem Volumen 2 abzulassen. Das Fluid ist bevorzugt Umgebungsluft. Zwischen der Innenwand 14 und der Außenwand 15 sind eine Vielzahl zugkraftübertragender Elemente 7 ausgebildet, die im dargestellten Ausführungsbeispiel als Stege ausgebildet sind. Es besteht auch die Möglichkeit, statt der Stege Gurte, Stifte oder auch nur Verbindungspunkte zwischen der Innenwand 14 und der Außenwand 15 vorzusehen. Im nicht mit Fluid befüllten Zustand kann die Innenwand 14 an der Außenwand 15 anliegen.

Der Hüllkörper 1 ist im Längsschnitt U-förmig ausgebildet, im Querschnitt senkrecht zu der Längserstreckung des Hüllkörpers 1 weist dieser eine im Wesentlichen kreisförmige, geschlossene Kontur auf. Im dargestellten, nicht befüllten Zustand ist somit ein Innenumfang 4 und ein Außenumfang 5 vorhanden, die Abmessungen der Umfänge 4, 5 ergeben sich aus der Formgebung, der Dimensionierung und den Materialeigenschaften.

Wird durch den Anschluss 3 Fluid in das abgeschlossene Volumen 2 eingeleitet, erhöht sich der Druck innerhalb des Volumens 2, da sowohl die Innenwand 14 als auch die Außenwand 15 aus einem elastischen Material hergestellt sind, vorzugsweise aus einem Elastomer oder einer Verbindung aus elastischen Abschnitten und unelastischen Abschnitten. Durch den erhöhten Innendruck wirken entsprechende Kräfte auf die Innenwand 14 und die Außenwand 15, wobei aufgrund der größeren Fläche der Außenwand 15 dort größere Kräfte wirken. Bei gleichen Materialeigenschaften hinsichtlich der Dehnung, beispielsweise wenn die Innenwand 14 und die Außenwand 15 aus dem gleichen Material bestehen, ergibt sich hieraus, dass aufgrund der größeren Kräfte auf die Außenwand 15 eine größere Verformung und Dehnung stattfindet, was dazu führt, dass insgesamt der Hüllkörper ausgedehnt wird. Dies ist in der rechten Darstellung gezeigt. Der Außenumfang 5'im druckbeaufschlagten Zustand ist im Vergleich zur linken Darstellung vergrößert. Auch der Innenumfang 4' ist im Vergleich zur Ausgangsstellung, die in der Strichlinie dargestellt ist, vergrößert, ebenso wie der Abstand zwischen der Innenwand 14 und der Außenwand 15.

Durch die Druckbeaufschlagung über das Ventil und den Zugang 3 ist es möglich, den Innendurchmesser und damit auch den Umfang 4 des Aufnahmeraums des Hüllkörpers 1 für die Gliedmaße oder den Stumpf zu vergrößern, so dass der nicht dargestellte Stumpf leicht eingeführt werden kann. Wird das Ventil an dem Zugang 3 geöffnet, entweicht Luft aus dem Volumen 2 aufgrund der elastischen Rückstellkräfte, die durch die Innenwand 14 und die Außenwand 15 bereitgestellt werden. Das Volumen des Aufnahmeraumes für den Stumpf verkleinert sich, die Innenwand 14 legt sich an den nicht dargestellten Stumpf an und es wird ein fester Sitz des Hüllkörpers 1 an dem Stumpf ermöglicht. Je nach Grad der Druckminderung ergibt sich eine angepasste Anpresskraft der Innenwand 14 an den Stumpf, wobei die maximale Anpresskraft dann erreicht ist, wenn das Volumen 2 minimiert wird.

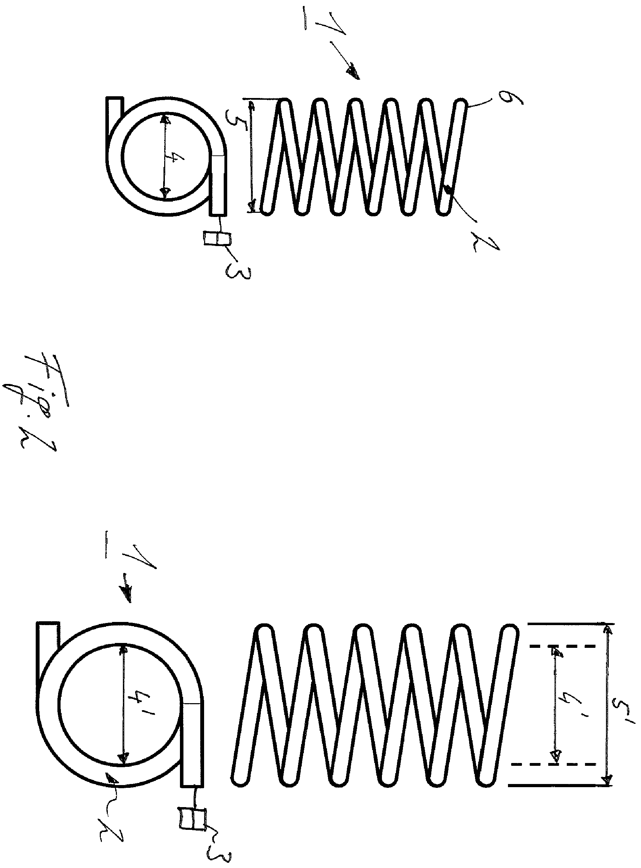

Figur 2 zeigt eine Variante der Erfindung, bei der statt eines hülsenartigen Volumens 2 ein schlauchartiges Volumen 2 durch einen schlauchförmigen Hohlraum 6 gebildet wird. Der Hohlraum 6 ist wendelförmig oder schraubenförmig angeordnet und ist in der linken Darstellung nicht mit einem Druckfluid befüllt und weist einen ersten Innenumfang 4 auf. Der Hohlraum 6 ist mehrfach gewickelt, so dass der Innenraum mehrfach vollständig umgeben wird.

In der rechten Darstellung wurde in das Volumen 2 Druckfluid gepumpt. Der Hohlkörper 6 hat aufgrund der Druckerhöhung die Tendenz, die Krümmung aufzuheben oder zu vergrößern, wodurch sich eine Durchmesservergrößerung und dadurch eine Vergrößerung des Innenumfangs 4 und des Außenumfangs 5 des Hüllkörpers 1 ergibt. In der rechten Darstellung der Figur 2 ist der vergrößerte Innenumfang 4'ebenso dargestellt wie der vergrößerte Außenumfang 5'. Der Hohlraum 6 in Gestalt der Wendel kann entweder den Hüllkörper 1 als Röhrchen ausbilden. Alternativ dazu kann ein solcher Hohlraum 6 in beispielsweise einem doppelwandigen Liner gemäß Figur 1 angeordnet sein oder aber auf der Innenseite und/oder Außenseite mit einer im Wesentlichen geschlossenen Schicht oder Wand ausgestattet sein, so dass eine glattflächige Anlage an der Gliedmaße oder dem Stumpf bzw. ein glattwandiger Abschluss auf der Außenseite erzielt werden kann. Der Hohlkörper 6 dient somit als Aktuator zur Durchmesservergrößerung und damit zur Vergrößerung des Innenumfanges 4 bei einer Druckerhöhung, so dass der Hüllkörper 1 im druckbeaufschlagten Zustand leicht angelegt werden kann. Bei einer Reduzierung des Innendruckes in dem Volumen 2 zieht sich der Hohlraum 6 wieder in die Ausgangsstellung gemäß der linken Darstellung zusammen, so dass der Hüllkörper 6 um den Stumpf oder um die Gliedmaße herum anliegt.

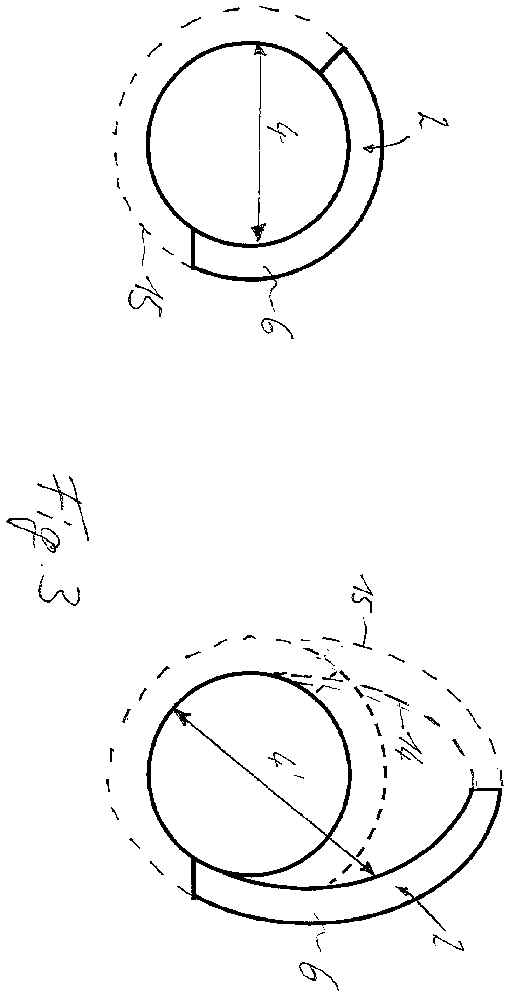

Figur 3 zeigt eine schematische Darstellung einer alternativen Ausgestaltung, bei der statt eines wendelförmig, umlaufenden Verlaufes des Hohlraumes 6 der nicht dargestellte Stumpf oder die nicht dargestellte Gliedmaße nur teilumfänglich von dem Hohlraum 6 umgeben wird. Auch hier kann das Volumen über ein Ventil und einen nicht dargestellten Anschluss mit einem veränderlichen Innendruck beaufschlagt werden. Gestrichelt ist eine Außenwand 15 dargestellt, die optional um den Hohlraum 6 herum angeordnet werden kann; auch einen Innenwand 4 kann an dem Hohlraum 6 angeordnet sein. Es können mehrere Hohlräume 6 axial hintereinander, also senkrecht zur Bildebene angeordnet sein. Die Hohlräume 6 können versetzt zueinander angeordnet sein, insbesondere in Umfangsrichtung zueinander versetzt, so dass sich der Effekt, der sich einstellt, wenn das Volumen 2 an dem Hohlraum 6 erhöht wird, einstellt, über die gesamte Länge in Axialrichtung erzielt werden kann.

In der rechten Darstellung der Figur 3 ist die Position und Form des Hohlraumes 6 nach der Druckerhöhung gezeigt. Der Hohlraum 6 neigt zu einer Begradigung und somit Vergrößerung des Krümmungsradiusses, wodurch sich die in der rechten Darstellung gezeigte Form des Hohlraumes 6 ergibt. Zusammen mit dieser Formänderung geht eine Vergrößerung des Innenumfanges 4'einher, die optional mit dem Hohlraum 6 gekoppelten Innen- und -Außenwände 14, 15 verformen sich dementsprechend, so dass sich eine angenähert elliptische oder ovale Innenkontur ergibt. Werden mehrere Hohlräume 6 in Axialrichtung hintereinander angeordnet, ergibt sich eine angenähert kreisförmige Innen- und Außenkontur. Sofern mehrere, fingerartige Hohlräume 6 vorgesehen sind, die in Axialrichtung zueinander beabstandet entlang der Längserstreckung des Hüllkörpers 1 angeordnet sind, können diese über eine gemeinsame Versorgungsleitung mit dem Druckfluid versorgt sein, so dass durch Betätigung nur eines Ventils die Möglichkeit besteht, den gesamten Hüllkörper 1 aufzuweiten oder in seinem Innenumfang 4 zu verringern.

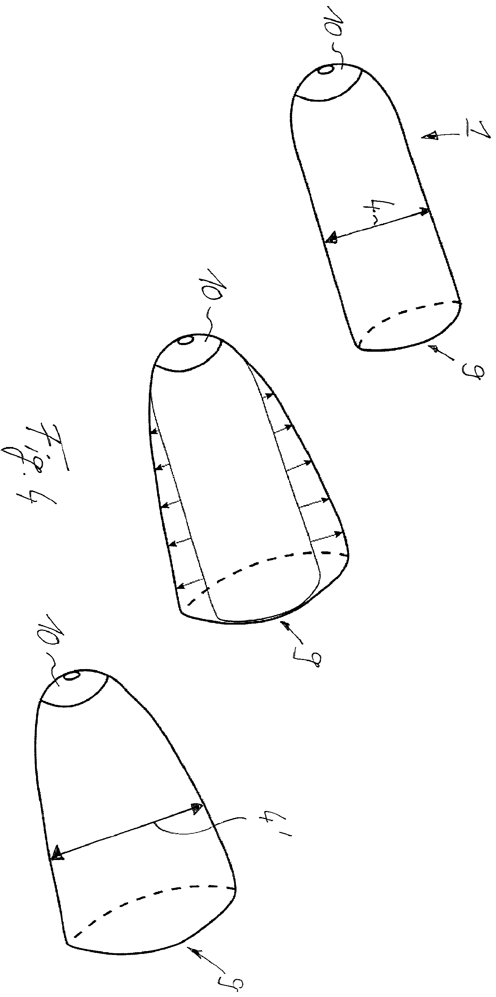

Figur 4 zeigt eine Ausführungsform des Hüllkörpers 1 in Gestalt eines Prothesenliners mit einer proximalen Einführöffnung 9 und einem geschlossenen distalen Endbereich 10. An dem distalen Endbereich 10 können eine formstabile Kappe sowie Aufnahmeeinrichtungen für mechanische Verriegelungselemente oder dergleichen angeordnet sein. Der Prothesenliner 1 weist in der oberen Darstellung einen ersten Innenumfang 4 auf. Von dem distalen Endbereich 10 erstreckt sich eine Seitenwand im Wesentlichen konisch in Richtung auf die proximale Einstiegsöffnung 9. Die Seitenwand oder der Seitenwandbereich ist doppelwandig ausgebildet, der distale Endbereich 10 ist entweder einlagig ausgebildet oder die beiden Schichten eines doppelwandigen Prothesenliners sind miteinander verklebt oder stoffschlüssig miteinander verbunden. In dem distalen Endbereich 10 kann kein Druckfluid eingeleitet werden.

Die mittlere Darstellung der Figur 4 zeigt die wirksamen Kräfte beim Einleiten eines Druckfluids in den in der Seitenwand ausgebildeten Hohlraum oder in das Volumen, das zwischen einem Innenliner und einem Außenliner eingeschlossen ist. In der rechten Darstellung ist die Form des Prothesenliners 1 bei einem erhöhten Volumeninnendruck dargestellt. Der Innenumfang 4'ist größer als der Ausgangsinnenumfang 4, die Einstiegsöffnung 9 des im distalen Endbereich 10 geschlossenen Prothesenliners ist im Vergleich zur ursprünglichen Einstiegsöffnung 9 wesentlich vergrößert. Dadurch wird das Einsteigen in den Prothesenliner 1 erleichtert. Nachdem das Stumpfende Kontakt mit der Innenseite des distalen Endbereiches 10 aufgenommen hat, wird das Ventil an dem Anschluss 3 geöffnet, die Luft entweicht und der Prothesenliner legt sich mit der Seitenwand an den Stumpf an.

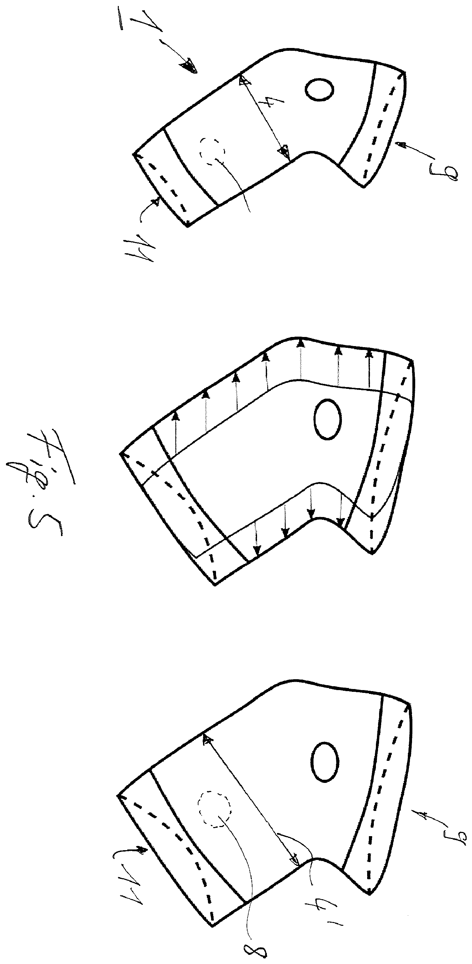

Eine nicht von der Erfindung umfasste Ausführungsform ist in Figur 5 dargestellt, bei der der Hüllkörper 1 in Gestalt einer pneumatisch veränderlichen Bandage ausgestaltet ist. Die Bandage kann als Kniebandage ausgebildet sein und ein Gelenk überbrücken. Alternativ ist die Ausgestaltung als Ellenbogenbandage, Handgelenksbandage oder Knöchelbandage möglich. Darüber hinaus kann die Bandage auch nicht gelenkübergreifend ausgebildet sein und beispielsweise den Oberschenkel, Oberarm, Unterschenkel oder Unterarm vollumfänglich umgreifen. Der Querschnitt der Bandage ist im dargestellten Ausführungsbeispiel geschlossen. Über die gesamte Länge der Bandage 1, die sich zwischen der proximalen Einstiegsöffnung 9 und der distalen Durchstiegsöffnung 11 erstreckt, ist sie doppelwandig ausgebildet und kann über einen nicht dargestellten Anschluss mit Druckluft beaufschlagt werden. Die Druckluftbeaufschlagung ist in der mittleren Darstellung der Figur 5 gezeigt, in der angedeutet ist, dass aus dem in der linken Darstellung gezeigten Ausgangszustand die Bandage 1 in den in der rechten Darstellung gezeigten Endzustand überführt wird. Der Innenumfang 4'in dem druckbeaufschlagten Zustand gemäß der rechten Darstellung ist größer als der Ausgangsinnenumfang 4, so dass die Bandage einfach angelegt werden kann. Eine Kompression findet durch Ablassen von Druckluft statt. An der Innenoberfläche der Bandage 1 können Bereiche 8 mit einer erhöhten Haftfähigkeit ausgebildet sein, so dass der Hüllköper 1 auf seiner Innenoberfläche 8 zumindest teilweise eine haftende Beschichtung aufweist.

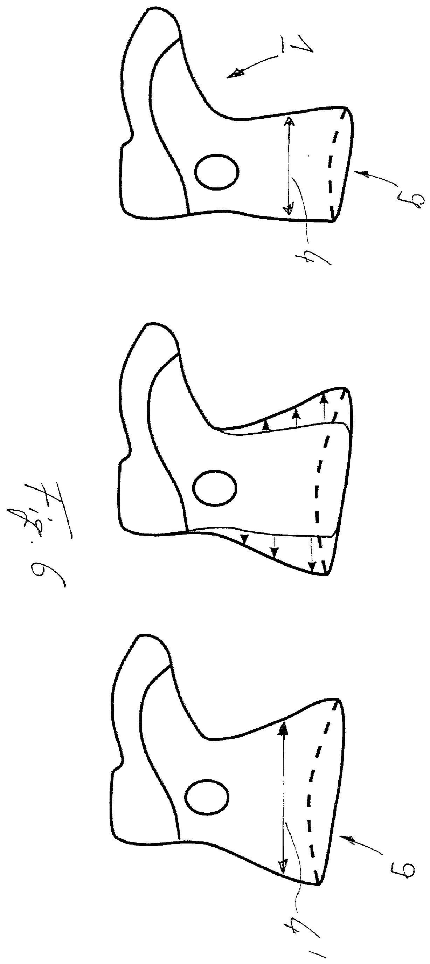

Figur 6 zeigt eine weitere, nicht von der Erfindung umfasste Ausführungsform, bei der Hüllkörper 1 als pneumatisch betriebener Schuh oder Stiefel ausgebildet ist. Der Schaftbereich des Hüllkörpers 1, der sich oberhalb des natürlichen Knöchelgelenks erstreckt, ist doppelwandig ausgebildet und bildet ein hülsenartiges Volumen aus, das mit Druckluft befüllt werden kann. Der Befüllvorgang ist in der mittleren Darstellung gezeigt, in der rechten Darstellung ist zu erkennen, dass die proximale Einstiegsöffnung 9 im Vergleich zur Ausgangsausdehnung wesentlich vergrößert ist, ebenso ist der Innenumfang 4'vergrößert zum ursprünglichen Innenumfang 4, so dass das Einsteigen in den Schuh oder Stiefel 1 wesentlich erleichtert wird. Nach dem Einsteigen kann der Druck in dem Volumen reduziert werden, wodurch sich der Schaft an den Unterschenkel oder Unterschenkelstumpf oder auch Unterschenkelschaft anlegt.

## Patentansprüche

1. Hüllkörper zur zumindest teilweisen Umhüllung einer Gliedmaße mit einem eingeschlossenen Volumen (2) und einem Anschluss (3) zum Zuführen und Abführen von Fluid in das Volumen (2) oder aus dem Volumen (2), wobei der Hüllkörper (1) einen Innenumfang (4, 4') und einen Außenumfang (5, 5') ausbildet, wobei der Hüllkörper (1) als Prothesenliner ausgebildet ist, einen Aufnahmeraum für einen Stumpf ausbildet und der Innenumfang (4, 4') des Prothesenliners (1) sich bei einer Druckerhöhung des Volumens vergrößert, wobei der Hüllkörper (1) eine Innenwand (14) und eine Außenwand (15) aufweist, die das Volumen (2) einschließen und dass zwischen der Innenwand (14) und der Außenwand (15) zugkraftübertragende Elemente (7) angeordnet sind, wobei sich das Volumen des Aufnahmeraumes bei einer Druckminderung verringert und die Innenwand (14) im angelegten Zustand eine Anpresskraft auf den Stumpf ausübt, **dadurch gekennzeichnet, dass** die maximale Anpresskraft erreicht ist, wenn das Volumen (2) minimal ist.

2. Hüllkörper nach Anspruch 1, **dadurch gekennzeichnet, dass** die Innenwand (14) und/oder Außenwand (15) faltbar und/oder elastisch ausgebildet ist.

3. Hüllkörper nach Anspruch 1, **dadurch gekennzeichnet, dass** die zugkraftübertragenden Elemente (7) zugstarr oder elastisch ausgebildet sind.

4. Hüllkörper nach einem der voranstehenden Ansprüche, **dadurch gekennzeichnet, dass** der Hüllkörper (1) einen zumindest teilumfänglich um den Innenumfang (4, 4') herumgelegten Hohlraum (6) aufweist.

5. Hüllkörper nach Anspruch 4, **dadurch gekennzeichnet, dass** der Hohlraum (6) zumindest teilkreisförmig oder wendelförmig um den Innenumfang (4, 4') herumgelegt ist.

6. Hüllkörper nach Anspruch 4 oder 5, **dadurch gekennzeichnet, dass** der Hohlraum (6) schlauchartig ausgebildet ist.

7. Hüllkörper nach Anspruch 6, **dadurch gekennzeichnet, dass** der Hohlraum (6) als elastischer Schlauch ausgebildet ist.

8. Hüllkörper nach einem der Ansprüche 4 bis 7, **dadurch gekennzeichnet, dass** der Hohlraum (6) an einer Wand (14, 15) anliegt oder zwischen einer Innenwand (14) und einer Außenwand (15) angeordnet ist.

9. Hüllkörper nach einem der voranstehenden Ansprüche, **dadurch gekennzeichnet, dass** der Hüllkörper (1) eine zumindest teilweise haftende Innenoberfläche (8) aufweist.

## Claims

1. An enveloping body for at least partly enveloping a limb, comprising an enclosed volume (2) and a connection (3) for supplying fluid to the volume (2) and discharging it from the volume (2), wherein the enveloping body (1) defines an inner circumference (4, 4') and an outer circumference (5, 5'), whereas the enveloping body (1) is configured as a prosthesis liner, establishing a receiving space for a stump and that the inner circumference (4, 4') of the prosthesis liner (1) increases as the pressure of the volume increases, whereas the enveloping body (1) has an inner wall (14) and an outer wall (15), which enclose the volume (2) and that elements (7) transmitting tensile force are arranged between the inner wall (14) and the outer wall (15), wherein the volume of the receiving space is reduced when the pressure is reduced and the inner wall (14) exerts a contact pressure on the stump in the applied state, **characterized in that** the maximum contact pressure is reached when the volume (2) is minimal.

2. The enveloping body as claimed in claim 1, **characterized in that** the inner wall (14) and/or outer wall (15) is foldable and/or elastic.

3. The enveloping body as claimed in claim 1, **characterized in that** the elements (7) transmitting tensile force are rigid under tension or elastic.

4. The enveloping body as claimed in one of the preceding claims,
**characterized in that** the enveloping body (1) has a hollow space (6) routed at least partially circumferentially about the inner circumference (4, 4').

5. The enveloping body as claimed in claim 4, **characterized in that** the hollow body (6) is routed in an at least partial circular shape or helical shape about the inner circumference (4, 4').

6. The enveloping body as claimed in claim 4 or 5, **characterized in that** the hollow space (6) has a hose-like configuration.

7. The enveloping body as claimed in claim 6, **characterized in that** the hollow space (6) is configured as an elastic hose.

8. The enveloping body as claimed in one of claims 4 through 7, **characterized in that** the hollow space (6) bears on a wall (14, 15) or is arranged between an inner wall (14) and an outer wall (15).

9. The enveloping body as claimed in one of the preceding claims,
**characterized in that** the enveloping body (1) has an at least partially adhesive inner surface (8).

## Revendications

1. Corps enveloppant destiné à envelopper au moins partiellement un membre, présentant un volume (2) enfermé et un raccord (3) pour l'amenée et l'évacuation de fluide dans le volume (2) ou hors du volume (2), le corps enveloppant (1) formant un pourtour intérieur (4, 4') et un pourtour extérieur (5, 5'), le corps enveloppant (1) étant conçu comme une doublure prothétique et formant un espace de réception pour un moignon, et le pourtour intérieur (4, 4') de la doublure prothétique (1) s'agrandissant lors d'une augmentation de pression du volume,
dans lequel
le corps enveloppant (1) présente une paroi intérieure (14) et une paroi extérieure (15) qui enferment le volume (2), et des éléments (7) transmettant des forces de traction sont disposés entre la paroi intérieure (14) et la paroi extérieure (15), le volume de l'espace de réception diminue lors d'une diminution de la pression, et, à l'état appliqué, la paroi intérieure (14) exerce une force de pression sur le moignon,
**caractérisé en ce que** la force de pression maximale est atteinte lorsque le volume (2) est minimal.

2. Corps enveloppant selon la revendication 1,
**caractérisé en ce que** la paroi intérieure (14) et/ou la paroi extérieure (15) est conçue de manière pliable et/ou élastique.

3. Corps enveloppant selon la revendication 1,
**caractérisé en ce que** les éléments (7) transmettant des forces de traction sont conçus de manière rigide en traction ou élastique.

4. Corps enveloppant selon l'une des revendications précédentes,
**caractérisé en ce que** le corps enveloppant (1) présente un espace creux (5) disposé au moins partiellement autour du pourtour intérieur (4, 4').

5. Corps enveloppant selon la revendication 4,
**caractérisé en ce que** l'espace creux (6) est disposé au moins en forme de cercle partiel ou en forme d'hélice autour du pourtour intérieur (4, 4').

6. Corps enveloppant selon la revendication 4 ou 5,
**caractérisé en ce que** l'espace creux (6) est conçu en forme de tuyau.

7. Corps enveloppant selon la revendication 6,
**caractérisé en ce que** l'espace creux (6) est conçu comme un tuyau élastique.

8. Corps enveloppant selon l'une des revendications 4 à 7,
**caractérisé en ce que** l'espace creux (6) est adjacent à une paroi (14, 15) ou est disposé entre une paroi intérieure (14) et une paroi extérieure (15).

9. Corps enveloppant selon l'une des revendications précédentes,
**caractérisé en ce que** le corps enveloppant (1) présente une surface intérieure (8) au moins partiellement adhérente.
